# EUROPÄISCHE PATENTSCHRIFT

(11) **EP 1 864 647 B1**
(45) Veröffentlichungstag und Bekanntmachung des Hinweises auf die Patenterteilung: **16.11.2011**
(21) Anmeldenummer: 07109380.1
(22) Anmeldetag: 01.06.2007
(51) Int. Cl.: A61K 8/41, A61Q 17/00

(54) **Acyclische Merocyanine in kosmetischen Zubereitungen**
Acyclical merocyanines in cosmetic preparations
Mérocyanine acyclique dans des préparations cosmétiques

(30) Priorität: 09.06.2006 DE 102006027238
(43) Veröffentlichungstag der Anmeldung: 12.12.2007
(73) Patentinhaber: Beiersdorf AG, 20253 Hamburg (DE)
(72) Erfinder: Skubsch, Kerstin, 25421, Pinneberg (DE); Mundt, Claudia, 28207, Bremen (DE); Wolber, Rainer, 22397, Hamburg (DE); Blatt, Thomas, 21379, Wedel (DE); Smuda, Christoph, 25474, Bönningstedt (DE); Reinecke, Myriam, 22529, Hamburg (DE)

(56) Entgegenhaltungen:
- EP-A2- 0 087 060
- WO-A-2004/006878
- WO-A-2006/003094
- WO-A-2007/068707
- WO-A-2007/071584

## Beschreibung

Die vorliegende Erfindung betrifft eine kosmetische Zubereitung mit einem acyclischen Merocyanin und mindestens einem Hautbefeuchtungsmittel.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Um der Haut auch ohne Sonnenstrahlung vorübergehend einen Braunton zu verschaffen, kann man sie nach dem Stand der Technik mit gefärbten (dekorativen) Schminkprodukten (meist mit Pigmenten auf Eisenoxid-Basis) oder mit so genannten "Selbstbräunern" wie Dihydroxyaceton (DHA) einfärben. Derartige Hautfärbungen haben jedoch eine Reihe von Nachteilen. Hautfärbungen mittels dekorativer Schminkprodukte (d.h. mit Farbstoffen wie Eisenoxiden) wirken häufig maskenhaft und färben leicht auf Kleidungsstücke ab. Hautbräunungen mittels DHA oder anderen "Selbstbräunern" führen zu einer Hautbräunung, welche die Haut nicht vor den schädlichen Einflüssen der UV-Strahlung schützt, wie es bei der natürlichen Bräunung (durch Sonnenstrahlen) der Fall ist.

Den Wunsch nach einem gesunden Aussehen und einer sportlich braunen Haut hegt der Mensch insbesondere dann, wenn er in seiner Freizeit mit anderen Menschen in Kontakt kommt. Einer der wohl beliebtesten Freizeitaktivitäten insbesondere junger Erwachsender, stellt der abendliche Besuch einer Diskothek dar. In Diskotheken wird zur Beleuchtung der Tanzfläche häufig so genanntes "Schwarzlicht" eingesetzt, welches Licht mit einer Wellenlänge von ungefähr 366 nm als Komponente des Spektrums enthält. Derartiges Licht führt bei unbehandelter Haut jedoch zu einem kränklich-bleichen Aussehen. Hautpartien, die mit dekorativen Kosmetika braun gefärbt wurden erscheinen besonders maskenhaft. Auch der Braunton, welcher durch Selbstbräuner auf der Haut erzeugt wurde kommt nicht richtig zur Geltung sondern wirkt eher aschenfarben.

Eine Vielzahl von Tagespflegeprodukten für das Gesicht enthält, ebenso wie Sonnenschutzmittel, zum Schutz vor Sonnenstrahlen UV-Lichtschutzfiltersubstanzen. Ein Teil dieser Lichtschutzfiltersubstanzen, beispielsweise Phenylen-1,4-bis-(2-benzimidazyl)-3,3'-5,5'-tetrasulfonsäuresalze haben jedoch den Nachteil bei "Schwarzlicht" zu fluoreszieren, so dass dem Tagespflege- oder Sonnenschutzmittel benutzenden Verbraucher abends in der Diskothek ein geisterhaftes Erscheinungsbild verliehen wird, wenn er das Hautpflegeprodukt nicht vorher entfernt hat. Auch wenn sich dieser Effekt bei einigen Nachtschwärmern großer Beliebtheit erfreut, so wird er von dem überwiegenden Teil der Verbraucher als unattraktiv empfunden.

Es war daher die Aufgabe der vorliegenden Erfindung, die Mängel des Standes der Technik zu beseitigen. Insbesondere war es die Aufgabe der vorliegenden Erfindung ein Kosmetikum zu entwickeln, welches der Haut im faden Lichtschein einer Diskothek (dem so genannten "Schwarzlicht") optisch eine gesunde sportlich braune Hautfärbung verleiht. Auch war es die Aufgabe der vorliegenden Erfindung ein Sonnenschutzmittel zu entwickeln, welches die Haut bei Sonnenschein wirkungsvoll vor den schädlichen Einflüssen der UV-Strahlung schützt, andererseits dazu führt, dass die Haut bei abendlichen Freizeitaktivitäten wie dem Diskobesuch als gesund gebräunt wahrgenommen wird.

Überraschend gelöst werden die Aufgaben durch eine kosmetische Zubereitung enthaltend eine Kombination aus der Verbindung 1 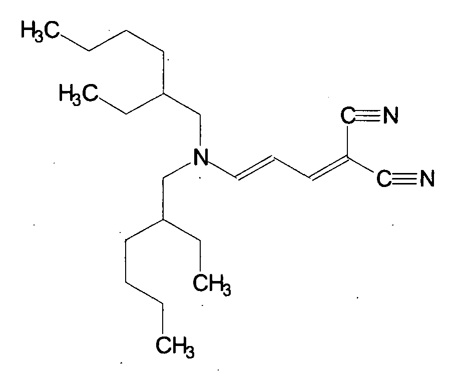 und einem oder mehreren Hautbefeuchtungsmitteln.

Die erfindungsgemäßen Zubereitungen zeigen überraschenderweise darüber hinaus eine erhöhte Photostabilität bei Lichtbestrahlung. Insbesondere Zubereitungen, welche photolabile Inhaltstoffe wie 4-(tert.-Butyl)-4'-methoxydibenzoylmethan enthalten, zeigen bei der Bestrahlung über einen längeren Zeitraum einen deutlich geringeren Abfall der Lichtschutzfilterwirkung als Zubereitungen ohne den erfindungsgemäßen Lichtschutzfilter. Die Kombination mit weiteren Lichtschutzfiltern führt im Regelfall zu einem überadditiven Anstieg des Lichtschutzfaktors (SPF) und zu einer hohen UV-A-Balance. Hautpartien, die mit der erfindungsgemäßen kosmetischen Zubereitung behandelt wurden, zeigen nach der Bestrahlung mit UV-Strahlen eine deutlich höhere Vitalität, als Hautpartien, die mit vergleichbaren Zubereitungen behandelt wurden, welche den erfindungsgemäßen Lichtschutzfilter nicht enthielten.

Es ist erfindungsgemäß vorteilhaft, wenn die erfindungsgemäße Zubereitung die Verbindung 1 in einer Konzentration 10 bis 0,1 Gewichts-%, und besonders bevorzugt in einer Konzentration von 5 bis 0,5 Gewichts-%, jeweils bezogen auf das Gesamtgewicht der Zubereitung, enthalten.

Es ist erfindungsgemäß bevorzugt, wenn die erfindungsgemäße Zubereitung die Verbindung 1 in einer Konzentration von 50 bis 0,1 Gewichts-% und ein oder mehrere Hautbefeuchtungsmittel in einer Gesamtkonzentration von 15 bis 0,5 Gewichts-%, jeweils bezogen auf das Gesamtgewicht der Zubereitung, enthalten.

Dabei ist es vorteilhaft im Sinne der vorliegenden Erfindung, wenn das Gewichtsverhältnis von Verbindung 1 zur Gesamtmenge an Hautbefeuchtungsmitteln von 3 : 1 bis 1:20 und bevorzugt von 1:1 bis 1:10 beträgt.

Erfindungsgemäß vorteilhafte Ausführungsformen der vorliegenden Erfindung sind dadurch gekennzeichnet, dass der oder die Hautbefeuchtungsmittel gewählt werden aus der Gruppe der Verbindungen Glycerin, Milchsäure und/oder Lactate, Butylenglykol, Propylenglykol, Biosaccaride Gum-1, Glycine Soja, Ethylhexyloxyglycerin, Pyrrolidoncarbonsäure, Harnstoff, gelierbare Polysaccharide wie Hyaluronsäure, Chitosan, Propylenglykol, 2-Methylpropan-1,3-diol, Ethylenglykol, Ethylenglykolmonoethyl- oder -monobutylether, Propylenglykolmonomethyl, -monoethyl-oder -monobutylether, Diethylenglykolmonomethyl- oder -monoethylether. Auch das fucosereiche Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist, kann erfindungsgemäß vorteilhaft eingesetzt werden.

Dabei ist es erfindungsgemäß bevorzugt, wenn der oder die Hautbefeuchtungsmittel gewählt werden aus der Gruppe der Verbindungen Glycerin, Methylpropandiol, Propandiol, Butylenglycol, 1,6 Hexandiol.

Die erfindungsgemäße Zubereitung kann neben der erfindungsgemäßen Verbindung 1 erfindungsgemäß vorteilhaft weitere UV-Lichtschutzfilter enthalten. So ist es erfindungsgemäß bevorzugt, wenn die erfindungsgemäße kosmetische Zubereitung einen oder mehrere weitere UV-Filter enthält, gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol) (Handelsname: Tinosorb M); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher ; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenylacrylat; Dimethicodiethylbenzalmalonat; 3-(4-(2,2-bis Ethoxy-carbonylvinyl)-phenoxy)propenyl)-methoxysiloxan / Dimethylsiloxan - Copolymer (INCI : Polysilicone-15; 2,4-Bis-{[4-(2-ethyl-hexyloxy)-2-hydroxy]-phenyl}-6-(4-methoxyphenyl)-1,3,5-triazin (INCI :Bis-Ethylhexyloxyphenol Methoxyphenyl Triazin); Dioctylbutylamidotriazon (INCI: Diethylhexyl-Butamidotriazone); 2,4-bis-[5-1 (dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyitriimino)-tris-benzoesäure-tris(2-ethylhexylester) (auch: 2,4,6-Tris-[anilino-(p-carbo-2'-ethyl-1'-hexyloxy)]-1,3,5-triazin (INCl: Ethylhexyl Triazone); 2,4,6-Tribiphenyl-4-yl-1,3,5-triazin; den Merocyaninverbindungen 1 bis 4, welche in der DE102005059741.6 offenbart sind;Titiandioxid; Zinkoxid in einer Konzentration von 0,01 bis 40 Gewichts-% bezogen auf das Gesamtgewicht der Zubereitung. Dabei sind Konzentrationen von 1 bis 20 Gewichts-%, bezogen auf das Gesamtgewicht der Zubereitung erfindungsgemäß bevorzugt.

Es ist erfindungsgemäß vorteilhaft, wenn die erfindungsgemäße Zubereitung frei ist von p-Methylbenzylidencampher.

Die Pigmente (Titandioxid, Zinkoxid) können vorteilhaft im Sinne der vorliegenden Erfindung auch in Form kommerziell erhältlicher öliger oder wässriger Vordispersionen zur Anwendung kommen. Diesen Vordispersionen können vorteilhaft Dispergierhilfsmittel und/oder Solubilisationsvermittler zugesetzt sein.

Die Pigmente (Titandioxid, Zinkoxid) können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), Bariumsulfat (Ba-SO₄) oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß vorteilhafte Ausführungsformen der vorliegenden Erfindung sind dadurch gekennzeichnet, dass die Zubereitung einen oder mehrere Wirkstoffe gewählt aus der Gruppe der Verbindungen Vitamin E und/oder dessen Derivate, Vitamin und/oder dessen Derivate, alpha-Liponsäure, Folsäure, Phytoen, D-Biotin, Coenzym Q10, alpha-Glucosylrutin, Carnitin, Carnosin, natürliche und/oder synthetische Isoflavonoide, Kreatin, Kreatinin, Taurin, β-Alanin, 8-Hexadecen-1,16-dicarbonsäure, Licochalcon A, Niacinamid, Panthenol, Dihydroxyaceton in einer Konzentration von 0,001-30 Gewichts-% pro Wirkstoff enthält.

Die bevorzugte Konzentration pro Einzelwirkstoff beträgt 0,05 bis 20 Gew.-% und insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Ferner vorteilhaft können die erfindungsgemäßen Zubereitungen auch Repellentien zum Schutz vor Mücken, Zecken und Spinnen und dergleichen enthalten. Vorteilhaft sind z. B. N,N-Diethyl-3-methylbenzamid (Handelsbezeichnung: Meta-delphene, "DEET"), Dimethylphtalat (Handelsbezeichnung: Palatinol M, DMP), 1-Piperidincarbonsäure-2-(2-hydroxyethyl)-1-methylpropylester sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9) und /oder Talkum.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind solche mit der INCI-Bezeichnung Acrylates/C10-30 Alkyl Acrylate Crosspolymer (z. B. Pemulen TR 1, Pemulen TR 2, Carbopol 1328 von der Fa. NOVEON) sowie Aristoflex AVC (INCI : Ammonium Acryloyldimethyltaurate/VP Copolymer).

Erfindungsgemäß vorteilhaft enthält die erfindungsgemäße Zubereitung Filmbildner. Filmbildner im Sinne der vorliegenden Erfindung sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet er nach dem Verdunsten des Lösemittels einen Film aus, der im wesentlichen dazu dient, die Lichtfilter auf der Haut zu fixieren und so die Wasserfestigkeit des Produktes zu steigern.

Es ist insbesondere von Vorteil, die Filmbildner aus der Gruppe der Polymere auf Basis von Polyvinylpyrrolidon (PVP) 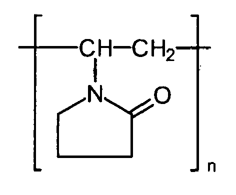 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole Acrylat/Octylacralymid Copolymer (Dermacryl 79). Ebenfalls vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

Die Ölphase der erfindungsgemäßen Zubereitung wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Jojobaöl Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether *(Cetiol OE)* und/oder Dicaprylylcarbonat, beispielsweise das unter der Handelsbezeichnung *Cetiol* CC bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon *(HallstarAB)* und/oder Diethylhexylnaphthalat *(Hallbrite TQ oder Corapan TQ von Symrise).*

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Die erfindungsgemäßen Zubereitungen können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:
(a) Siloxanelastomere, welche die Einheiten R₂SiO und RSiO_{1,5} und/oder R₃SiO_{0,5} und/oder SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind, wobei die verwendeten Mengenateile so gewählt werden, dass die Menge des Wasserstoffes des Organopolysiloxans (1) oder der ungesättigten aliphatischen Gruppen des Organopolysiloxans (2)
   - im Bereich von 1 bis 20 mol-% liegt, wenn das Organopolysiloxan nicht zyklisch ist und
   - im Bereich von 1 bis 50 mol-% liegt, wenn das Organopolysiloxan zyklisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCI-Bezeichnung Dimethicone / Vinyl Dimethicone Crosspolymer, beispielsweise das von DOW CORNING unter der Handelsbezeichnungen DOW CORNING 9506 Powder erhältliche.

Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Die erfindungsgemäße Zubereitung enthält erfindungsgemäß bevorzugt Parfümstoffe.

Die kosmetischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Substanzen zum Verhindern oder Steigern des Schäumens, Farbstoffe, Pigmente, die eine färbende Wirkung haben, Schaumstabilisatoren, Elektrolyte, organische Lösungsmittel oder Silikonderivate.

Vorteilhaft im Sinne der vorliegenden Erfindung sind Zubereitungen zur Pflege der Haut: sie können dem kosmetischen Lichtschutz, ferner als Schminkprodukt in der dekorativen Kosmetik dienen.

Entsprechend ihrem Aufbau können kosmetische Zusammensetzungen im Sinne der vorliegenden Erfindung, beispielsweise verwendet werden als Hautschutzcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Es ist auch vorteilhaft im Sinne der vorliegenden Erfindung, kosmetische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescremes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische Zubereitungen, die in der Form eines Sonnenschutzmittels vorliegen.

Erfindungsgemäß ist insbesondere die Verwendung der erfindungsgemäßen Zubereitung zum Schutz vor Hautalterung (insbesondere zum Schutz vor UV-bedingter Hautalterung) sowie als Sonnenschutzmittel.

Erfindungsgemäß vorteilhaft weist die erfindungsgemäße Zubereitung einen pH-Wert von 5 bis 8 auf. Dieser kann durch die herkömmlichen Säuren, Basen und Puffersysteme eingestellt werden.

Zur Anwendung werden die erfindungsgemäßen kosmetischen Zubereitungen in der für Kosmetika üblichen Weise auf die Haut und/oder die Haare in ausreichender Menge aufgebracht.

Es ist erfindungsgemäß vorteilhaft, wenn die erfindungsgemäße Zubereitung in Form eines Gels, einer Emulsion, einer Dispersion oder eines Öls vorliegt.

Erfindungsgemäß ist die Verwendung der Kombination aus Verbindung 1 und Hautbefeuchtungsmitteln in kosmetischen Zubereitungen um der Haut ein gebräuntes Aussehen unter der Bestrahlung von Licht mit der Wellenlänge von etwa 366 nm (Disko-Licht, "Schwarzlicht") zu verleihen.

Erfindungsgemäß ist die Verwendung der Verbindung 1 in kosmetischen Zubereitungen zur Verbesserung des natürlichen, gebräunten Teints in der Disco (insbesondere bei 366 nm).

Erfindungsgemäß ist die Verwendung der Kombination aus Verbindung 1 und Hautbefeuchtungsmitteln in kosmetischen Sprays (insbesondere Sonnenschutzmitteln).

Erfindungsgemäß ist die Verwendung der Kombination aus Verbindung 1 und Hautbefeuchtungsmitteln in kosmetischen Zubereitungen (insbesondere Sonnenschutzmitteln), die für die Tränkung kosmetischer Tücher vorgesehen sind.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**Beispiele für Gele**

| | **1** | **2** | **3** | **4** | **5** | **6** |
|---|---|---|---|---|---|---|
| Acrylates/Octylacrylamide Copolymer | 1,0 | 1,0 | 1,0 | 1,0 | 1,0 | 1,0 |
| Alohol Denat. | 50,0 | 62,0 | 59,2 | 70,0 | 70,0 | 69,0 |
| Butyl Methoxydibenzoylmethan | 4,5 | 4,5 | | | 2,5 | 4,5 |
| Butylene Glycol Dicaprylat/Dicaprat | | | 7,5 | | | 2 |
| C12-15 Alkyl Benzoate | 5,0 | 8,5 | 5,0 | 2 | 7,5 | |
| Phenylethylbenzoat | 3,0 | | | 2,5 | | |
| Cocoglycerid | | | | | | 2 |
| Tridecylsalicylat | 2 | 1,5 | | | 3 | 1 |
| Diethylamino Hydroxybenzoyl Hexyl Benzoate | | | 4,5 | 3,5 | | |
| Ethylhexyl Methoxycinnamat | 5 | 9,5 | | 6,5 | 6,5 | 9,5 |
| Ethylhexyl Salicylat | 4,5 | 4,5 | 4,5 | 4,5 | | |
| Homosalat | | | | | | 4,5 |
| Hydroxypropylcellulose | 2 | 0,8 | 1 | 0,8 | 0,5 | 0,8 |
| Octocrylen | 9,5 | 4,8 | 9,5 | 4,3 | 3,8 | |
| Ethylhexyltriazin | | | | | 2 | |
| Benzophenon-3 | 3 | | | | | |
| Drometrizol Trisiloxan | | | | 0,5 | 1,0 | |
| Verbindung 1 | 0,5 | 1,5 | 2,5 | 5 | 3,5 | 0,1 |
| Bis-Ethylhexyloxyphenol Methoxy¬phenyltriazin | | | | | | 1 |
| Vitamin E Acetat | | | | 0,5 | | 0,2 |
| Glycerin | 5 | | 3 | | | 2 |
| Propandiol | | 10 | 1 | 2 | 4 | |
| Parfüm, Farbstoffe | q.s. | q.s. | q.s. | q.s. | q.s. | q.s. |
| Wasser | ad 100 | ad 100 | ad 100 | ad 100 | ad 100 | ad 100 |

**Beispiele für Sprays**

| | **1** | **2** | **3** | **4** | **5** | **6** |
|---|---|---|---|---|---|---|
| Acrylat/Octylacrylamid Copolymer | 1 | | | | 1 | 1 |
| VP/VA Copolymer | | 1,0 | | 0,5 | | |
| Alkohol Denat. | 42 | 57 | 60 | 53 | 35,5 | 43,5 |
| Bis-Ethylhexyloxyphenol Methoxyphenyl Triazin | | | | | 1,0 | |
| Uvasorb® K2A | 0,5 | | 1,0 | | | 3,0 |
| Butyl Methoxydibenzoylmethan | 4,5 | 2 | | 3 | 4,5 | |
| Cyclomethicon | 4,9 | 2 | 5 | 0,5 | 8,0 | 8 |
| Diethylamino Hydroxybenzoyl Hexyl Benzoat | | 2 | 3 | | | 4,5 |
| Ethylhexyl Methoxycinnamat | 9,5 | | | 7 | 6,5 | 9,5 |
| Ethylhexyl Salicylat | 4,5 | 3,5 | 2 | 4 | 4,5 | 2,5 |
| Drometrizol Trisiloxan | | 0,5 | | | | 2,0 |
| Tridecylsalicylat | 0,5 | 2,5 | 4 | 7 | 10 | 3 |
| Homosalat | 9,5 | 5 | 3 | | 9,5 | 6,5 |
| Octocrylen | 9,5 | | 8 | | 7,5 | 9,5 |
| Verbindung 1 | 0,5 | 2,5 | 6,8 | 1 | 2 | 4 |
| Merocyanin | | | 2 | | | |
| Butylen Glycol Dicaprylat/Dicaprat | | 9 | | | | |
| C12-15 Alkyl Benzoat | | 2 | | 2 | | |
| Phenylbenzoat | | | 7 | | 4 | |
| Diethylhexylnaphthalat | | 6 | | 3 | | |
| Isopropyl Lauroyl Sarkosinat | 2 | | 1 | | 3 | |
| Phenyl Trimethicone | 2 5 | | | | 1 2 | |
| Octyldodekanol | | | | 8 | | |
| Glycerin | 5 | 4 | | 5 | 10 | 5 |
| Butylenglycol | | | 4 | | | |
| Methylpropandiol | | | | 3 | | 1 |
| Vitamin E Acetat | 0,1 | | 0,5 | | | |
| Parfüm, Farbstoffe | q.s. | q.s. | q.s. | q.s. | q.s. | q.s. |
| Wasser | ad 100 | ad 100 | ad 100 | ad 100 | ad 100 | ad 100 |

**O/W-Emulsionen**

| | **1** | **2** | **3** | **4** | **5** | **6** |
|---|---|---|---|---|---|---|
| Glyceryl Stearat Citrat | 2 | 2 | 3 | | | |
| Glyceryl Stearat SE | | | | 1 | 1 | 1,5 |
| Cetearyl Alkohol + PEG-40 Rizinusöl+ Natrium Cetearyl Sulfat | | | | 2,5 | 2,5 | 3 |
| Cetearylalkohol | | | 1 | 1 | | |
| Stearylalkohol | 0,5 | | | | | 2 |
| Myristylmyristat | 1,0 | 1 | | | 3 | |
| Acrylates/C₁₀₋₃₀ Alkyl Acrylat Cross-polymer | 0,1 | 0,2 | | | 0,1 | |
| Carbomer | | 0,2 | 0,3 | 0,2 | | |
| Xanthan Gum | 0,4 | | 0,2 | 0,2 | 0,3 | 0,4 |
| C₁₂₋₁₅ Alkyl Benzoat | | 3 | | | 5 | |
| 2-Phenylethylbenzoat | 5 | 2 | | | | |
| Butylenglycol Dicaprylat/Dicaprat | 5 | | | | 3 | 3 |
| Tridecylsalicylat | 1,5 | 2,5 | 0,25 | 5,9 | 7 | 15 |
| Dicaprylcaprat | 2 | 2 | | | 2 | 2 |
| Cyclomethicon | | | 5 | 10 | | |
| Dimethicon | | | | 5 | | |
| PVP Hexadecen Copolymer | | 0,5 | | | | 1 |
| Propylenglykol | | | 1 | | 5 | 3 |
| Glycerin | 7,5 | | 7 | 10 | 13 | 3 |
| 1,6 Hexandiol | | 5 | | 2 | | 1 |
| Alkohol denat. | 2 | 3 | | 7 | | |
| Titandioxid | 3 | | | 2 | | |
| Merocyanin | 2 | | 2 | | 3 | 3 |
| Phenylen-1,4-bis-(2-benzimidazyl)-3,3'-5,5'-tetrasulfonsäuresalz | 3 | 2 | | | | |
| Ethylhexyltriazin | 2,5 | 2 | | 1 | | |
| Phenylbenzimidazol Sulfonsäure | | | 2 | | | 1 |
| 4-Methoxyzimtsäure(2-ethylhexyl)ester | 9,5 | 5 | | 2 | | |
| Butyl Methoxydibenzoylmethan | | 1 | | | 3 | |
| Ethylhexylsalicylat | 2 | | 0,5 | 4 | 9,5 | |
| Octocrylen | | | | | 5 | 7 |
| Bis-Ethylhexyloxyphenol Methoxy- | | 1 | | 1 | | 1 |
| phenyltriazin | | | | | | |
| Verbindung 1 | 2 | 3 | 4 | 1 | 0,25 | 5 |
| Vitamin E Acetat | 0,2 | 0,2 | 0,2 | 0,3 | 0,1 | 0,5 |
| Na₂H₂EDTA | 0,1 | 0,1 | 0,2 | 0,2 | 0,2 | 0,5 |
| Parfüm, Konservierungsmittel | q.s. | q.s. | q.s. | q.s. | q.s. | q.s. |
| Farbstoffe, usw. | q.s. | q.s. | q.s. | q.s. | q.s. | q.s. |
| Citronensäure, Natriumcitrat | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100,0 | ad 100,0 | ad 100,0 | ad 100,0 | ad 100,0 |

**W/O-Emulsionen**

| | **1** | **2** | **3** | **4** | **5** | **6** |
|---|---|---|---|---|---|---|
| Polyglyceryl-2 Dipolyhydroxystearat | 3 | 5 | 3 | | | |
| PEG-30 Dipolyhydroxystearat | | | 2 | 3 | 4 | 5 |
| Natrium-Stärke Octenylsuccinat | 0,5 | 0,4 | 0,6 | 0,3 | 0,5 | 1 |
| Glycin | 0,3 | 0,3 | 0,5 | 0,4 | | |
| Alkohol | 2 | 5 | 2 | 0,5 | 8 | 1 |
| Magnesiumsulfat | 0,2 | 0,3 | 0,3 | 0,4 | 0,5 | 0,2 |
| C₁₂₋₁₅ Alkyl Benzoat | 5 | 3 | | | 5 | |
| Triheptanoin | | 2 | | | | |
| Butylenglycol Dicaprylat/Dicaprat | 5 | | | | 3 | 3 |
| Dicaprylyl Ether | | | | | 2 | |
| Mineralöl | | 4 | | 6 | | 8 |
| Octyldodecanol | 2 | | | | | |
| Dicaprylcaprat | | 2 | | | 2 | 2 |
| Cyclomethicon | 5 | | 5 | 10 | | |
| Dimethicon | | | | 5 | | |
| Isohexadecan | | 1 | | | | |
| Methylpropandiol | 5 | 8 | | | 3 | 3 |
| Glycerin | 3 | 5 | 7 | 10 | | 3 |
| Tridecylsalicylat | | 1 | 2 | | | 0,5 |
| Ethylhexylmethoxycinnamat | 5 | 7 | 5 | | | |
| Ethylhexyltriazin | 2 | 3 | 3 | | | 3 |
| Diethylhexylbutamidotriazin | | 1,5 | 1,5 | | | 1,5 |
| Butyl Methoxydibenzoylmethan | | 4 | | | | |
| 2-Phenylethylbenzoat | | | 2 | | 4 | |
| Isopropyl Lauroyl Sarkosinat | | | 1 | | 2 | |
| 2-(4'-Diethylamino-2'-hydoxybenzoyl)-benzoesäurehexylester | | | | 2 | | |
| Verbindung 1 | 1 | 2 | 4 | 0,75 | 5 | 2,5 |
| Titandioxid | 5 | 4 | 2 | | 3 | 4 |
| Bis-Ethylhexyloxyphenol Methoxyphenyltriazin | 1 | 2 | 2 | | 2 | 3 |
| Methylen Bis-Benzotriazolyl Tetramethylbutylphenol | 2 | | 3 | | 1 | |
| Merocyanin | 2 | | 2 | 5 | 1 | |
| Vitamin E Acetat | 0,2 | 0,2 | 0,2 | 0,3 | 0,1 | 0,5 |
| Na₂H₂EDTA | 0,1 | 0,1 | 0,2 | 0,2 | 0,2 | 0,5 |
| Parfüm, Konservierungsmittel | q.s. | q.s. | q.s. | q.s. | q.s. | q.s. |
| Wasser | ad 100,0 | ad 100,0 | ad 100,0 | ad 100,0 | ad 100,0 | ad 100,0 |

**Hydrodispersionen**

| | **1** | **2** | **3** | **4** | **5** | **6** |
|---|---|---|---|---|---|---|
| Glyceryl Stearat Citrat | | 0,40 | | | | |
| Sodium Carbomer | | | | | 0,30 | |
| Acrylates/C10-30 Alkyl Acrylate Cross-polymer | | | 0,30 | 0,40 | 0,10 | 0,10 |
| Ceteareth-20 | | | 1,00 | | | |
| Xanthan Gummi | 0,50 | | | 0,15 | | 0,50 |
| Dimethicon / Vinyl Dimethicon Cross-polymer | | | | 5,00 | | 3,00 |
| 2-(4'-Diethylamino-2'-hydoxybenzoyl)-benzoesäurehexylester | 0,25 | | | 0,50 | 2,00 | 1,50 |
| Butyl Methoxydibenzoylmethan | 2 | | 3,50 | | | |
| Bis-Ethylhexyloxyphenol Methoxypheny Triazin | | 2,00 | | 0,25 | | |
| Dinatrium Phenyl Dibenzimidazol Tetrasulfonat | 0,75 | | | | | 1,00 |
| Phenylbenzimidazol Sulfonsäure | | | 2,00 | | | |
| Ethylhexyl Methoxycinnamat | | | 7,00 | | 5,00 | 8,00 |
| Diethylhexyl Butamido Triazin | | | 2,00 | 2,00 | | |
| Ethylhexyl Triazin | 4,00 | 3,00 | | | 4,00 | |
| Octocrylen | | | | 10,00 | | 2,50 |
| Titandioxid | 0,50 | 2,00 | 1,00 | 2,00 | 3,00 | 1,00 |
| Verbindung 1 | 1 | 2 | 4 | 0,75 | 5 | 2,5 |
| Drometrizol Trisiloxan | | | | 1 | | 0,5 |
| Terephthaliden Dicampher Sulfonsäure | | | | 0,5 | | 0,75 |
| C₁₂₋₁₅ Alkyl Benzoat | 2,00 | | 2,50 | | | |
| Butylenglycol Dicaprylat/Dicaprat | 4,00 | | | | 6,00 | |
| Dicaprylyl Carbonat | | 3,00 | | | | |
| Dicaprylylether | | 2,00 | | | | |
| Cyclomethicon | | | | 7,50 | | |
| 2-Phenylethylbenzoat | | | 4 | | 2 | |
| Diethylhexylnaphthalat | 5 | | 6 | | | |
| Tridecylsalicylat | 2 | 3 | 1 | 5 | 3 | 0,5 |
| PVP Hexadecen Copolymer | 0,50 | | 0,50 | | 0,50 | 1,00 |
| Glycerin | 10,00 | 5,00 | 5,00 | | 5,00 | 15,00 |
| Propandiol | | 2,00 | | 5,00 | | |
| Butylenglycol | | 7,00 | | | | |
| Glycin Soja | | | | 1,00 | | |
| Vitamin E Acetat | 0,50 | 0,25 | 0,50 | 0,25 | 0,75 | 1,00 |
| α - Glycosylrutin | | | | | 0,25 | |
| Trinatrium EDTA | | 1,00 | 1,00 | 0,10 | 0,20 | |
| Ethanol | 3,00 | 10,00 | 4,00 | 3,50 | 0,5 | 1,00 |
| Konservierungsmittel | q.s. | q.s. | q.s. | q.s. | q.s. | q.s. |
| Parfüm, Farbstoffe, | q.s. | q.s. | q.s. | q.s. | q.s. | q.s. |
| Wasser | ad 100 | ad 100 | ad 100 | ad 100 | ad 100 | ad 100 |

**kosmetische Schäume**

| **Emulsion** | **A** | **B** | **C** |
|---|---|---|---|
| Stearinsäure | 2 | 2 | |
| Palmitinsäure | | | 1,5 |
| Cetylalkohol | 2,5 | 2 | |
| Stearylalkohol | | | 3 |
| PEG-100 Stearat | | | 3,5 |
| PEG-40 Stearat | | 2 | |
| PEG-20-Stearat | 3 | | |
| Sorbitanstearat | | 0,8 | |
| C₁₂₋₁₅ Alkyl Benzoat | 5 | | |
| C₁₂₋₁₃ Alkyl Tartrat | | | 7 |
| Butylenglycol Dicaprylat/Dicaprat | | 6 | |
| Dicaprylyl Ether | | | 2 |
| Cyclomethicon | | 2 | 3 |
| Butylenglycol | 1 | | |
| Isohexadecan | 2 | | |
| Methylpropandiol | | 1 | |
| Propylenglykol | | | 5 |
| Glycerin | 5 | 7 | |
| 2,4-bis-[5-1 (dimethylpropyl)benzoxazol-2-yl-(4-phenyl)-imino]-6-(2-ethylhexyl)-imino-1,3,5-triazin | | | 2 |
| 2-(4'-Diethylamino-2'-hydoxybenzoyl)-benzoesäurehexylester | 2 | | |
| Dimethicodiethylbenzalmalonat | | 3 | |
| Homosalat | | 5 | |
| Phenylbenzimidazol Sulfonsäure | | 2 | 2 |
| Benzophenon-3 | 2 | | |
| Octylsalicylat | | 5 | |
| Octocrylen | 2 | | |
| Bis-Ethylhexyloxyphenol Methoxyphenyltriazin | | 3 | |
| 2,2'-Methylen-bis-(6-(2 H-benzotriazol-2-yl)-4-(1,1,3,3-tetramethylbutyl)-phenol) | | | 8 |
| 2,4,6-Tris-(biphenyl)-1,3,5-triazin | 5 | | |
| Verbindung 1 | 3 | 6 | 10 |
| C8-C16 Alkylpolyglycosid | 1 | | |
| Vitamin E Acetat | 0,6 | 0,5 | 0,2 |
| Kreatin/Kreatinin | | | 0,5 |
| BHT | | | 0,1 |
| Na₂H₂EDTA | 0,50 | | |
| Parfüm, Konservierungsmittel | q.s. | q.s. | q.s. |
| Farbstoffe, usw. | q.s. | q.s. | q.s. |
| Natriumhydroxid | q.s. | | q.s. |
| Kaliumhydroxid | | q.s. | |
| Wasser | ad 100,0 | ad 100,0 | ad 100,0 |

## Patentansprüche

1. Kosmetische Zubereitung enthaltend eine Kombination aus der Verbindung 1 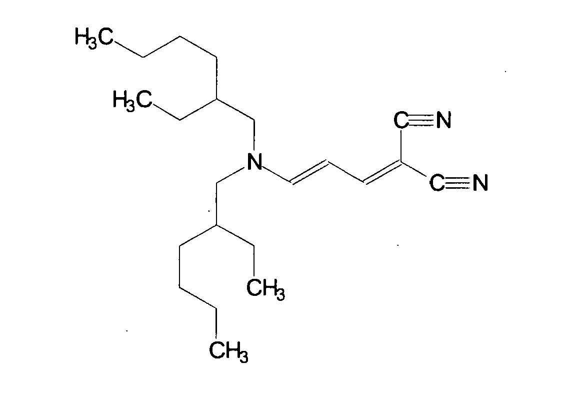 Verbindung 1 und einem oder mehreren Hautbefeuchtungsmitteln.

2. Kosmetische Zubereitung nach Anspruch 1, **dadurch gekennzeichnet, dass** die Zubereitung die Verbindung 1 in einer Konzentration von 0,1 bis 10 Gewichts-% und ein oder mehrere Hautbefeuchtungsmittel in einer Gesamtkonzentration von 0,1 bis 50 Gewichts-%, jeweils bezogen auf das Gesamtgewicht der Zubereitung, enthalten.

3. Kosmetische Zubereitung nach einem der vorhergehenden Ansprüche, **dadurch gekennzeichnet, dass** das Gewichtsverhältnis von Verbindung 1 zur Gesamtmenge an Hautbefeuchtungsmitteln von 3:1 bis 1:20 beträgt.

4. Kosmetische Zubereitung nach einem der vorhergehenden Ansprüche, **dadurch gekennzeichnet, dass** der oder die Hautbefeuchtungsmittel gewählt werden aus der Gruppe der Verbindungen Glycerin, Milchsäure und/oder Lactate, Butylenglykol, Propylenglykol, Biosaccaride Gum-1, Glycine Soja, Hydroxyethylharnstoff, Ethylhexyloxyglycerin, Pyrrolidoncarbonsäure, Harnstoff, gelierbare Polysaccharide wie Hyaluronsäure, Chitosan, Propylenglykol, 2-Methylpropan-1,3-diol, Ethylenglykol, Ethylenglykolmonoethyl- oder-monobutylether, Propylenglykolmonomethyl, -monoethyl- oder -monobutylether, Diethylenglykolmonomethyl- oder -monoethylether.

5. Kosmetische Zubereitung nach einem der vorhergehenden Ansprüche, **dadurch gekennzeichnet, dass** der oder die Hautbefeuchtungsmittel gewählt werden aus der Gruppe der Verbindungen Glycerin, Methylpropandiol, Propandiol, Butylenglycol, 1,6 Hexandiol.

6. Kosmetische Zubereitung nach einem der vorhergehenden Ansprüche, **dadurch gekennzeichnet, dass** die Zubereitung einen oder mehrere weitere UV-Filter enthält, gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bomylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenyl-acrylat; Dimethicodiethylbenzalmalonat; 3-(4-(2,2-bis Ethoxycarbonylvinyl)-phenoxy)propenyl)-methoxysiloxan / Dimethylsiloxan - Copolymer; 2,4-Bis-{[4-(2-ethyl-hexyloxy)-2-hydroxy]-phenyl}-6-(4-methoxyphenyl)-1,3,5-triazin (INCI :Bis-Ethylhexyloxyphenol Methoxyphenyl Triazin); Dioctylbutylamidotriazon (INCI: Diethylhexyl-Butamidotriazone); 2,4-bis-[5-1 (dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Titiandioxid; Zinkoxid in einer Konzentration von 0,01 bis 40 Gewichts-% bezogen auf das Gesamtgewicht der Zubereitung.

7. Kosmetische Zubereitung nach einem der vorhergehenden Ansprüche, **dadurch gekennzeichnet, dass** die Zubereitung einen oder mehrere Wirkstoffe gewählt aus der Gruppe der Verbindungen Vitamin E und/oder dessen Derivate, Vitamin und/oder dessen Derivate, alpha-Liponsäure, Folsäure, Phytoen, D-Biotin, Coenzym Q10, alpha-Glucosylrutin, Carnitin, Carnosin, natürliche und/oder synthetische Isoflavonoide, Kreatin, Kreatinin, Taurin, ß-Alanin, 8-Hexadecen-1,16-dicarbonsäure, Licochalcon A, Niacinamid, Panthenol, Dihydroxyaceton in einer Konzentration von 0,001-30 Gewichts-% pro Wirkstoff enthält.

8. Kosmetische Zubereitung nach einem der vorhergehenden Ansprüche, **dadurch gekennzeichnet, dass** die Zubereitung ein oder mehrere Verdickungsmittel gewählt aus der Gruppe Siliciumdioxid, Aluminiumsilikate, Polysaccharide wie Hyaluronsäure, Xanthangummi, Hydroxypropylmethylcellulose, Polyacrylate, enthält.

9. Kosmetische Zubereitung nach einem der vorhergehenden Ansprüche, **dadurch gekennzeichnet, dass** die Zubereitung in Form eines Gels, einer Emulsion, einer Dispersion oder eines Öls vorliegt.

10. Kosmetische Zubereitung nach einem der vorhergehenden Ansprüche, **dadurch gekennzeichnet, dass** die Zubereitung Parfümstoffe enthält.

## Claims

1. Cosmetic preparation comprising a combination of the compound 1 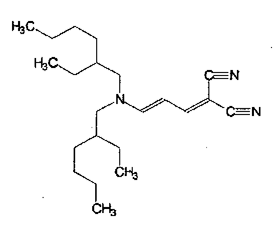 Compound 1 and one or more skin-moisturizing agents.

2. Cosmetic preparation according to Claim 1, **characterized in that** the preparation comprises the compound 1 in a concentration of from 0.1 to 10% by weight and one or more skin-moisturizing agents in a total concentration of from 0.1 to 50% by weight, in each case based on the total weight of the preparation.

3. Cosmetic preparation according to one of the preceding claims, **characterized in that** the weight ratio of compound 1 to the total amount of skin moisturizing agents is from 3:1 to 1:20

4. Cosmetic preparation according to one of the preceding claims, **characterized in that** the skin moisturizing agent or agents are selected from the group of the compounds glycerol, lactic acid and/or lactates, butylene glycol, propylene glycol, biosaccharide gum-1, glycine soya, hydroxyethylurea, ethylhexyloxyglycerol, pyrrolidonecarboxylic acid, urea, gelable polysaccharides such as hyaluronic acid, chitosan, propylene glycol, 2-methylpropane-1,3-diol, ethylene glycol, ethylene glycol monoethyl or monobutyl ether, propylene glycol monomethyl, monoethyl or monobutyl ether, diethylene glycol monomethyl or monoethyl ether.

5. Cosmetic preparation according to one of the preceding claims, **characterized in that** the skin moisturizing agent or agents are selected from the group of the compounds glycerol. Methylpropanediol, propanediol, butylene glycol, 1,6-hexanediol.

6. Cosmetic preparation according to one of the preceding claims, **characterized in that** the preparation comprises one or more further UV filters selected from the group of the compounds phenylene-1,4-bis(2-benzimidazyl)-3,3'-5,5'-tetrasulphonic acid salts; 2-phenylbenzimidazole-5-sulphonic acid salts; 1,4-di(Z-oxo-10-sulphv-3-bornylidenemethyl)benzene and salts thereof; 4-(2-oxo-3-bornylidenemethyl)benzenesulphonic acid salts; 2-methyl-5-(2-oxo-3-bornylidenemethyl)sulphonic acid salts: 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; terephthalidenedicamphorsulphonic acid; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; hexyl 2-(4'-diethylamino-2'-hydroxybenzoyl)benzoate, 4-(tert-butyl)-4'-methoxydibenzoylmethane; homomenthyl salicylate; 2-ethylhexyl 2-hydroxybenzoate; 2-ethylhexyl 2-cyano-3,3-diphenylacrylate; dimethicodiethyl benzalmalonate; 3-(4-(2,2-bis-ethoxycarbonylvinyl)phenoxy)propenyl)methoxy-siloxane/dimethylsiloxane copolymer; 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine); dioctylbutylamidotriazone (INCI: Diethylhexyl-Butamidotriazone); 2,4-bis[5-1(dimethylpropyl)-benzoxazol-2-yl-(4-phenyl)imino]-6-(2-ethylhexyl)imino-1,3,5-triazine with the CAS No. 288254-16-0; tris(2-ethylhexyl) 4,4',4"-(1,3,5-triazine-2,4,6-triyltriimino)trisbenzoate (also: 2,4,6-tris[aniline(p-carbo-2'-ethyl-1'-hexyloxy)]-1,3,5-triazine (INCI: Ethylhexyl Triazone); 2,4,6-tribiphenyl-4-yl-1,3,5-triazine; titanium dioxide; zinc oxide in a concentration of from 0.01 to 40% by weight, based on the total weight of the preparation.

7. Cosmetic preparation according to one of the preceding claims, **characterized in that** the preparation comprises one or more active ingredients selected from the group of the compounds vitamin E and/or derivatives thereof, vitamin A and/or derivatives thereof, alpha-lipoic acid, folic acid, phytoene, D-biotin, coenzyme Q10, alpha-glucosylrutin, carnitine, carnosine, natural and/or synthetic isoflavonoids, creatine, creatinine, taurine, β-alanine, 8-hexadecene-1,16-dicarboxylic acid, licochalcone A, niacinamide, panthenol, dihydroxyacetone in a concentration of from 0.001-30% by weight per active ingredient.

8. Cosmetic preparation according to one of the preceding claims, **characterized in that** the preparation comprises one or more thickeners selected from the group consisting of silicon dioxide, aluminium silicates, polysaccharides such as hyaluronic acid, xanthan gum, hydroxypropylmethylcellulose, polyacrylates.

9. Cosmetic preparation according to one of the preceding claims, **characterized in that** the preparation is present in the form of a gel, an emulsion, a dispersion or an oil.

10. Cosmetic preparation according to one of the preceding claims, **characterized in that** the preparation comprises perfume substances.

## Revendications

1. Composition cosmétique contenant une combinaison du composé 1 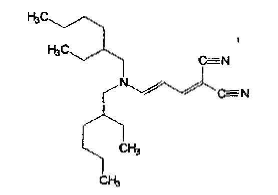 Composé 1,
et d'un ou de plusieurs agents d'hydratation de la peau.

2. Composition cosmétique selon la revendication 1, **caractérisée en ce que** la composition contient le composé 1 en une concentration de 0,1 à 10% en poids et un ou plusieurs agents d'hydratation de la peau en une concentration totale de 0,1 à 50% en poids, à chaque fois par rapport au poids total de la composition.

3. Composition cosmétique selon l'une quelconque des revendications précédentes, **caractérisée en ce que** le rapport pondéral du composé 1 à la quantité totale d'agents d'hydratation de la peau est de 3:1 à 1:20.

4. Composition cosmétique selon l'une quelconque des revendications précédentes, **caractérisée en ce que** le ou les agents d'hydratation de la peau sont choisis dans le groupe formé par les composés glycérol, acide lactique et/ou lactates, butylèneglycol, propylèneglycol, biosaccaride gum-1, glycine soja, hydroxyéthylurée, éthylhexyloxyglycérol, acide pyrrolidonecarboxylique, urée, polysaccharides gélifiables, tels que l'acide hyaluronique, chitosane, propylèneglycol, 2-méthylpropane-1,3-diol, éthylèneglycol, éthylèneglycolmonoéthyléther ou éthylèneglycolmonobutyléther, propylèneglycolmonométhyléther, propylèneglycolmonoéthyléther ou propylèneglycolmonobutyléther, diéthylèneglycolmonométhyléther ou diéthylèneglycolmonoéthyléther,

5. Composition cosmétique selon l'une quelconque des revendications précédentes, **caractérisée en ce que** le ou les agents d'hydratation de la peau sont choisis dans le groupe formé par les composés glycérol, méthylpropanediol, propanediol, butylèneglycol, 1,6-hexanediol.

6. Composition cosmétique selon l'une quelconque des revendications précédentes, **caractérisée en ce que** la composition contient un ou plusieurs autres filtres des UV, choisis dans le groupe formé par les composés sels de l'acide phénylène-1,4-bis-(2-benzimidazyl)-3,3'-5,5'-tétrasulfonique ; sels de l'acide 2-phénylbenzimidazole-5-sulfonique ; 1,4-di(2-oxo-10-sulfo-3-bornylidèneméthyl)-benzène et ses sels ; sels de l'acide 4-(2-oxo-3-bornylidèneméthyl)benzènesulfonique ; sels de l'acide 2-méthyl-5-(2-oxo-3-bornylidèneméthyl)sulfonique ; 2,2'-méthylénebis-(6-(2-H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-(4-méthylbenzylidène)camphre ; 3-benzylidénecamphre ; salicylate d'éthylhexyle ; acide téréphtalidènedicamphresulfonique ; ester 2-éthylhexylique de l'acide 4-(diméthylamino)-benzoïque ; ester amylique de l'acide 4-(diméthylamino)benzoïque ; ester di(2-éthylhexylique) de l'acide 4-méthoxybenzalmalonique ; ester 2-éthylhexylique de l'acide 4-méthoxycinnamique ; ester iso-amylique de l'acide 4-méthoxycinnamique ; 2-hydroxy-4-méthoxybenzophénone, 2-hydroxy-4-méthoxy-4'-méthylbenzophénone ; 2,2'-dihydroxy-4-méthoxybenzophénone ; ester hexylique de l'acide 2-(4'-diéthylamino-2'-hydroxybenzoyl)-benzoïque, 4-(tert-butyl)-4'-méthoxydibenzoylméthane ; salicylate d'homomenthyle ; 2-hydroxybenzoate de 2-éthylhexyle ; 2-cyano-3,3-diphénylacrylate de 2-éthylhexyle ; diméthicodiéthylbenzalmalonate ; copolymère de 3-(4-(2,2-bis-(éthoxycarbonylvinyl)-phénoxy)propényl)-méthoxysiloxane/diméthylsiloxane ; 2,4-bis-{[4-(2-éthylhexyloxy)-2-hydroxy]-phényl}-6-(4-méthoxyphényl)-1,3,5-triazine (INCI : Bis-Ethylhexyloxyphenol Methoxyphenyl Triazin) ; dioctylbutylamidotriazone (INCI Diethylhexyl-Butamidotriazone) ; 2,4-bis-[5-1(diméthylpropyl)benzoxazol-2-yl-(4-phényl)-imino]-6-(2-éthylhexyl)-imino-1,3,5-triazine (n° CAS 288254-16-0) ; ester tris(2-éthylhexylique) de l'acide 4,4',4"-(1,3,5-triazine-2,4,8-triyltriimino)-trisbenzoïque (également : 2,4,6-tris-[anilino-(p-carbo-2'-éthyl-1'-hexyloxy)]-1,3,5-triazine (INCI : Ethylhexyl Triazone) ; 2,4,6-tribiphényl-4-yl-1,3,5-triazine ; dioxyde de titane ; oxyde de zinc en une concentration de 0,01 à 40% en poids par rapport au poids total de la composition.

7. Composition cosmétique selon l'une quelconque des revendications précédentes, **caractérisée en ce que** la composition contient une ou plusieurs substances actives choisies dans le groupe formé par les composés vitamine E et/ou ses dérivés, vitamine A et/ou ses dérivés, acide alpha-liponique, acide folique, phytoéne, D-biotine, co-enzyme Q10, alpha-glucosylrutine, carnitine, carnosine, isoflavonoïdes naturels et/ou synthétiques, créatine, créatinine, taurine, ß-alanine, acide 8-hexadécène-1,16-dicarboxylique, licochalcone A, niacinamide, panthénol, dihydroxyacétone en une concentration de 0,001-30% en poids par substance active.

8. Composition cosmétique selon l'une quelconque des revendications précédentes, **caractérisée en ce que** la composition contient un ou plusieurs épaississants choisis dans le groupe formé par le dioxyde de silicium, les silicates d'aluminium, les polysaccharides tels que l'acide hyaluronique, la gomme de xanthane, l'hydroxypropylméthylcellulose, les polyacrylates.

9. Composition cosmétique selon l'une quelconque des revendications précédentes, **caractérisée en ce que** la composition se trouve sous forme d'un gel, d'une émulsion, d'une dispersion ou d'une huile.

10. Composition cosmétique selon l'une quelconque des revendications précédentes, **caractérisée en ce que** la composition contient des parfums.
